# EUROPEAN PATENT APPLICATION

(11) **EP 4 116 310 A1**
(43) Date of publication of application: **11.01.2023**
(21) Application number: 21765047.2
(22) Date of filing: 03.03.2021
(51) Int. Cl.: C07F 9/40, B01J 31/04, B01J 31/20, C07B 61/00, C07F 9/58

(54) **METHOD FOR MANUFACTURING PHOSPHONATE ESTERS AND METHOD FOR MANUFACTURING PHOSPHATE ESTERS**

(30) Priority: 04.03.2020 JP 2020036865
(71) Applicant: FUJIFILM Wako Pure Chemical Corporation, Osaka 540-8605 (JP)
(72) Inventor: KOBAYASHI Shu, Tokyo 113-0033 (JP); SAITO Yuki, Tokyo 113-0033 (JP)
(74) Representative: Winter, Brandl - Partnerschaft mbB
(86) International application number: PCT/JP2021/008113
(87) International publication number: WO 2021/177339

(57) **Abstract**

The present invention provides a method for efficiently manufacturing a phosphonate ester by phosphonylating an alcohol under mild conditions, and a method for manufacturing a phosphate ester. In the method for manufacturing a phosphonate ester of the present invention, a compound represented by the formula (1) is reacted with a compound represented by the formula (2) in the presence of a zinc catalyst to obtain a compound represented by the formula (3).

Formula (1) X-OH

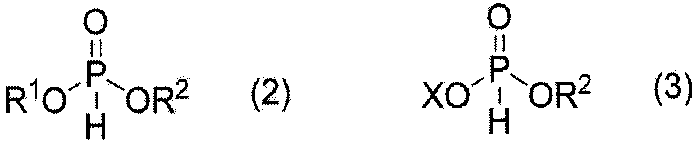

X represents an organic group. R¹ represents an alkyl group. R² represents an organic group.

## Description

### Technical Field

The present invention relates to a method for manufacturing a phosphonate ester and a method for manufacturing a phosphate ester.

### Background Art

Phosphorus-based compounds such as phosphonate esters are used for various purposes. As disclosed in Non-Patent Literature 1, a method for manufacturing a phosphonate ester by phosphonylating an alcohol in the presence of a Ti compound such as Ti(iPrO)₄ (where iPr represents an isopropyl group) is known as the method for manufacturing a phosphonate ester.

### Citation List

### Non-Patent Literature

Non-Patent Literature 1 Tetrahedron Letters, vol. 29, No.27, pp 3327 to 3330.

### Summary of Invention

### Technical Problem

On the other hand, from an industrial point of view, it is desirable that the phosphonate ester can be manufactured under milder conditions (lower than 100°C).

In a case where the present inventors carried out a reaction under mild conditions (lower than 100°C) using the Ti compound described in Non-Patent Literature 1, a desired compound could not be manufactured efficiently (with high yield).

The present invention has been made in view of the foregoing circumstances, and an object of the present invention is to provide a manufacturing method capable of efficiently manufacturing a phosphonate ester by phosphonylating an alcohol under mild conditions.

In addition, an object of the present invention is to provide a manufacturing method capable of manufacturing a phosphate ester using the phosphonate ester.

### Solution to Problem

As a result of extensive studies to achieve the foregoing objects, the present inventors have found that the foregoing objects can be achieved by the following configurations.

(1) A method for manufacturing a phosphonate ester, comprising reacting a compound represented by the formula (1) which will be described later with a compound represented by the formula (2) which will be described later in the presence of a Zinc catalyst to obtain a compound represented by the formula (3) which will be described later.
(2) The method according to (1), in which the Zinc catalyst comprises an oxygen-containing organic ligand.
(3) The method according to (2), in which the Zinc catalyst is a catalyst represented by the formula (A) which will be described later.
(4) The method according to any one of (1) to (3), in which the Zinc catalyst comprises a ligand represented by the formula (B) which will be described later or a carboxylate anion.
(5) The method according to any one of (1) to (4), in which the reaction is carried out while removing a compound represented by the formula (4) which will be described later, which is produced as a by-product, from a reaction system.
(6) The method according to any one of (1) to (5), in which a ratio of a molar amount of the Zinc catalyst used to a molar amount of the compound represented by the formula (1) used is 0.01 or more.
(7) The method according to any one of (1) to (6), in which the reaction is carried out in the presence of a solvent having a Fedors' solubility parameter of less than 11.21 (cal/cm³)^{½}.
(8) A method for manufacturing a phosphate ester, having a first step of reacting a compound represented by the formula (1) which will be described later with a compound represented by the formula (2) which will be described later in the presence of a Zinc catalyst to obtain a compound represented by the formula (3) which will be described later, and a second step of reacting the compound represented by the formula (3) which will be described later with a compound represented by the formula (8) which will be described later in the presence of an oxidizing agent to obtain a compound represented by the formula (9) which will be described later.
(9) The method for manufacturing a phosphate ester according to (8), in which the first step and the second step are carried out in one pot.

### Advantageous Effects of Invention

According to the present invention, it is possible to provide a manufacturing method capable of efficiently manufacturing a phosphonate ester by phosphonylating an alcohol under mild conditions.

In addition, according to the present invention, it is possible to provide a method capable of efficiently manufacturing a phosphate ester using the phosphonate ester.

### Description of Embodiments

Hereinafter, the present invention will be described in detail.

It should be noted that any numerical range represented by using "to" in the present specification means a range containing the numerical values before and after "to" as the lower limit value and the upper limit value, respectively.

One of the feature points of the manufacturing method of the present invention is that a Zn catalyst (zinc catalyst) is used. Using a Zn catalyst makes it possible to manufacture a desired compound efficiently (with high yield) under mild conditions. More specifically, in a case where the compound represented by the formula (1) which will be described later and the compound represented by the formula (2) which will be described later react with each other, the R¹O group in the formula (2) is eliminated to obtain a desired compound.

The manufacturing method of the present invention is a method for manufacturing a phosphonate ester, containing reacting a compound represented by the formula (1) which will be described later (hereinafter, also simply referred to as "compound 1") with a compound represented by the formula (2) which will be described later (hereinafter, also simply referred to as "compound 2") in the presence of a Zn catalyst to obtain a compound represented by the formula (3) which will be described later (hereinafter, also simply referred to as "compound 3").

In the following, first, the materials used in the present manufacturing method will be described in detail, and then the procedure of the manufacturing method will be described in detail.

### Compound represented by formula (1) (compound 1)

The compound 1 is an alcohol having an OH group.

Formula (1) X-OH

X represents an organic group.

The number of carbon atoms in the organic group is not particularly limited, and is preferably 1 to 30, more preferably 1 to 20, and still more preferably 1 to 10 from the viewpoint that the compound 3 can be manufactured more efficiently (hereinafter, also simply referred to as "the viewpoint that the effect of the present invention is more excellent").

The type of the organic group is not particularly limited, and examples thereof include a hydrocarbon group which may have a heteroatom. Examples of the heteroatom include an oxygen atom, a nitrogen atom, a sulfur atom, and a halogen atom. It should be noted that the organic group preferably does not have a hydroxy group.

Above all, from the viewpoint that the effect of the present invention is more excellent, the organic group is preferably an aliphatic hydrocarbon group which may have a substituent, an aromatic hydrocarbon group which may have a substituent, a heterocyclic group which may have a substituent, or a group in which two or more thereof are combined.

The number of carbon atoms in the aliphatic hydrocarbon group is not particularly limited, and is preferably 1 to 30, more preferably 1 to 20, and still more preferably 1 to 10 from the viewpoint that the effect of the present invention is more excellent.

The aliphatic hydrocarbon group may be linear or branched. In addition, the aliphatic hydrocarbon group may have a cyclic structure. The cyclic structure may be a monocyclic structure or a polycyclic structure.

Examples of the aliphatic hydrocarbon group include an alkyl group, an alkenyl group, an alkynyl group, and a group in which two or more thereof are combined (for example, a group represented by alkenyl group-alkylene group-^{∗} where ^{∗} represents a bonding position, or a group represented by alkynyl group-alkylene group-^{∗} where ^{∗} represents a bonding position).

The aliphatic hydrocarbon group may have a fluorine atom. Above all, from the viewpoint that the effect of the present invention is more excellent, the aliphatic hydrocarbon group which may have a fluorine atom is preferably an alkyl group which may have a fluorine atom.

In a case where the aliphatic hydrocarbon group has a fluorine atom, the number of fluorine atoms is not particularly limited, and is preferably 1 to 10, more preferably 1 to 5, and still more preferably 3 to 5 from the viewpoint that the effect of the present invention is more excellent.

In a case where the aliphatic hydrocarbon group has a fluorine atom, hydrogen atoms of the aliphatic hydrocarbon group may be partially substituted with fluorine atoms, or hydrogen atoms of the aliphatic hydrocarbon group may be completely substituted with fluorine atoms.

The aliphatic hydrocarbon group having a fluorine atom is preferably a group represented by the formula (10).

Formula (10) R⁵-L^{a}-^{∗}

R⁵ represents a perfluoroalkyl group. L^{a} represents an alkylene group. ^{∗} represents a bonding position.

The number of carbon atoms in the perfluoroalkyl group is not particularly limited, and is preferably 1 to 5, more preferably 1 to 3, and still more preferably 1 from the viewpoint that the effect of the present invention is more excellent.

The number of carbon atoms in the alkylene group is not particularly limited, and is preferably 1 to 5, more preferably 1 to 3, and still more preferably 1 from the viewpoint that the effect of the present invention is more excellent.

The number of carbon atoms in the aromatic hydrocarbon group is not particularly limited, and is preferably 6 to 18 and more preferably 6 to 12 from the viewpoint that the effect of the present invention is more excellent.

The aromatic hydrocarbon ring constituting the aromatic hydrocarbon group may have a monocyclic structure or a polycyclic structure.

Examples of the aromatic hydrocarbon ring constituting the aromatic hydrocarbon group include a benzene ring, a naphthalene ring, an anthracene ring, a phenanthrene ring, a fluorene ring, a triphenylene ring, a tetracene ring, and a pyrene ring.

Examples of the heterocyclic group include an aromatic heterocyclic group and an aliphatic heterocyclic group.

The number of carbon atoms in the heterocyclic group is not particularly limited, and is preferably 3 to 18 and more preferably 3 to 12 from the viewpoint that the effect of the present invention is more excellent.

Examples of the heteroatom other than the carbon atom constituting the heterocyclic group include an oxygen atom, a nitrogen atom, and a sulfur atom.

The heterocyclic ring constituting the heterocyclic group may have a monocyclic structure or a polycyclic structure.

Examples of the aromatic heterocyclic ring constituting the aromatic heterocyclic group include a pyridine ring, a pyrrole ring, an imidazole ring, a pyrazole ring, an oxazole ring, a thiazole ring, a triazole ring, a furan ring, a purine ring, a cytosine ring, an adenine ring, a guanine ring, a uracil ring, and a thiophene ring.

Examples of the aliphatic heterocyclic ring constituting the aliphatic heterocyclic group include an oxolane ring (tetrahydrofuran ring), a pyrrolidine ring, a thiolane ring (tetrahydrothiophene ring), a piperidine ring, an oxane ring (tetrahydropyran ring), a thiane ring (tetrahydrothiopyran ring), a piperazine ring, a morpholine ring, a quinuclidine ring, a pyrrolidine ring, an azetidine ring, an oxetane ring, an aziridine ring, and a dioxane ring.

Examples of the "group in which two or more thereof are combined" include a group in which an aliphatic hydrocarbon group and an aromatic hydrocarbon group are combined, and a group in which an aliphatic hydrocarbon group and a heterocyclic group are combined.

Examples of the group in which an aliphatic hydrocarbon group and an aromatic hydrocarbon group are combined include a group represented by the formula (6).

Formula (6) R³-L-^{∗}

R³ represents an aromatic hydrocarbon group which may have a substituent. L represents a divalent aliphatic hydrocarbon group. ^{∗} represents a bonding position.

The suitable aspect of the aromatic hydrocarbon group represented by R³ is as described above.

The number of carbon atoms in the divalent aliphatic hydrocarbon group represented by L is not particularly limited, and is preferably 1 to 30, more preferably 1 to 20, and still more preferably 1 to 10 from the viewpoint that the effect of the present invention is more excellent.

The divalent aliphatic hydrocarbon group may be linear or branched. In addition, the aliphatic hydrocarbon group may have a cyclic structure. The cyclic structure may be a monocyclic structure or a polycyclic structure.

Examples of the divalent aliphatic hydrocarbon group include an alkylene group, an alkenylene group, and an alkynylene group, among which the alkylene group is preferable.

Examples of the group in which an aliphatic hydrocarbon group and a heterocyclic group are combined include a group represented by the formula (7).

Formula (7) R⁴-L-^{∗}

R⁴ represents a heterocyclic group which may have a substituent. L represents a divalent aliphatic hydrocarbon group. ^{∗} represents a bonding position.

The suitable aspect of the heterocyclic group represented by R⁴ is as described above.

The definition of L is the same as the definition of L in the formula (6).

The aliphatic hydrocarbon group, the aromatic hydrocarbon group, and the heterocyclic group each may have a substituent.

In a case where the aliphatic hydrocarbon group, the aromatic hydrocarbon group, and the heterocyclic group each have a substituent, the number of substituents is not particularly limited and may be one or plural.

The type of the substituent is not particularly limited, and examples thereof include an alkyl group, an alkenyl group, an alkynyl group, an aryl group, an amino group, an alkoxy group, an aryloxy group, an aromatic heterocyclic oxy group, an acyl group, an alkoxycarbonyl group, an aryloxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, an aromatic heterocyclic thio group, a sulfonyl group, a sulfinyl group, a ureido group, a phosphate amide group, a mercapto group, a halogen atom (a fluorine atom, a chlorine atom, a bromine atom, or an iodine atom), a cyano group, a sulfo group, a carboxy group, a nitro group, a hydroxamic acid group, a sulfino group, a hydrazino group, an imino group, a heterocyclic group (for example, a heteroaryl group), a silyl group, a known protective group (for example, a protective group for an amino group; more specifically, a benzyloxymethyl group (BOM group), a tert-butoxycarbonyl group (Boc group)), and a group in which these groups are combined. More specific examples of the substituent include a benzyloxymethyl group (BOM group), a tert-butoxycarbonyl group (Boc group), and a group in which these groups are combined. It should be noted that the substituent may be further substituted with a substituent.

In a case where the aliphatic hydrocarbon group, the aromatic hydrocarbon group, and the heterocyclic group each have a plurality of substituents, the substituents may be bonded to each other to form a ring. For example, a group represented by the following formula (X) corresponds to an aspect in which two substituents of the oxolane ring are bonded to each other to form a ring. In the following formula (X), ^{∗} represents a bonding position and R^{b} represents a hydrogen atom or an organic group. The definition of the organic group represented by R^{b} is the same as the definition of the organic group represented by X described above.

The organic group represented by R^{b} is preferably a heterocyclic group which may have a substituent. The definition of the heterocyclic group is as described above. The type of the substituent that the heterocyclic group may have is not particularly limited, and examples thereof include the groups exemplified above. Above all, the organic group represented by R^{b} is preferably a group derived from a cytosine ring which may have a substituent (for example, a residue formed by removing one hydrogen atom from a cytosine ring which may have a substituent), a group derived from an adenine ring which may have a substituent (for example, a residue formed by removing one hydrogen atom from an adenine ring which may have a substituent), a group derived from a guanine ring which may have a substituent (for example, a residue formed by removing one hydrogen atom from a guanine ring which may have a substituent), or a group derived from a uracil ring which may have a substituent (for example, a residue formed by removing one hydrogen atom from a uracil ring which may have a substituent. It should be noted that examples of the group derived from a cytosine ring, the group derived from an adenine ring, the group derived from a guanine ring, and the group derived from a uracil ring include groups represented by the following formulae (Y1) to (Y4), respectively. In the formulae (Y1) to (Y4), R^{Y1} to R^{Y8} each independently represent a hydrogen atom or a substituent. Examples of the substituent include the groups exemplified above. In the formulae (Y1) to (Y4), ^{∗} represents a bonding position.

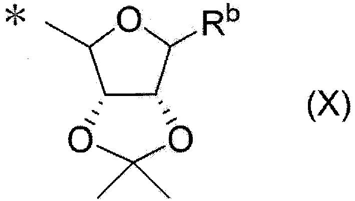

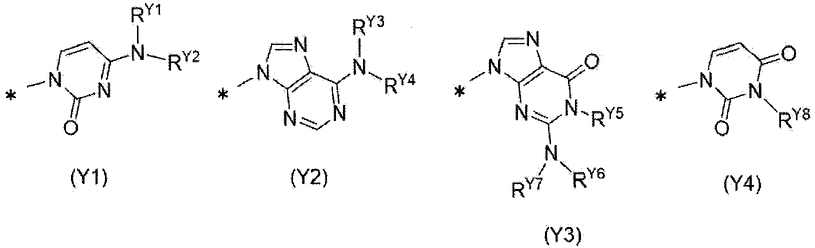

Above all, from the viewpoint that the effect of the present invention is more excellent, X is preferably an aliphatic hydrocarbon group which may have a substituent (for example, a group represented by the formula (X), or a fluorine atom), a group represented by the formula (6), or a group represented by the formula (7), and more preferably an alkyl group which may have a group represented by the formula (X), an alkyl group substituted with an alkenyl group, an alkyl group substituted with an alkynyl group, a group represented by the formula (6) in which L is an alkylene group, or a group represented by the formula (7) in which L is an alkylene group.

The aliphatic hydrocarbon group having a group represented by the formula (X) is preferably a group represented by the formula (11).

Formula (11) R⁶-L^{b}-^{∗}

R⁶ represents a group represented by the formula (X). L^{b} represents an alkylene group. ^{∗} represents a bonding position.

The number of carbon atoms in the alkylene group is not particularly limited, and is preferably 1 to 5, more preferably 1 to 3, and still more preferably 1 from the viewpoint that the effect of the present invention is more excellent.

### Compound represented by formula (2) (compound 2)

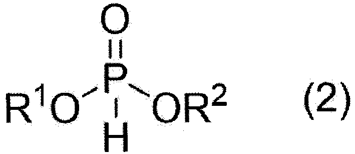

R¹ represents an alkyl group that may have a fluorine atom.

The number of carbon atoms in the alkyl group is not particularly limited, and is preferably 1 to 30, more preferably 1 to 20, still more preferably 1 to 10, and particularly preferably 1 to 4 from the viewpoint that the effect of the present invention is more excellent.

The alkyl group may be linear or branched. In addition, the alkyl group may have a cyclic structure. The cyclic structure may be a monocyclic structure or a polycyclic structure.

The alkyl group is preferably a methyl group, an ethyl group, a propyl group, or a butyl group, and more preferably a methyl group.

In a case where the alkyl group has a fluorine atom, the number of fluorine atoms is not particularly limited, and is preferably 1 to 10, more preferably 1 to 5, and still more preferably 3 to 5 from the viewpoint that the effect of the present invention is more excellent.

In a case where the alkyl group has a fluorine atom, hydrogen atoms of the alkyl group may be partially substituted with fluorine atoms, or hydrogen atoms of the alkyl group may be completely substituted with fluorine atoms.

The alkyl group having a fluorine atom is preferably the group represented by the formula (10).

R² represents an organic group.

The definition of the organic group represented by R² is the same as the definition of the organic group represented by X described above.

From the viewpoint that the effect of the present invention is more excellent, the organic group represented by R² is preferably an aliphatic hydrocarbon group which may have a substituent, an aromatic hydrocarbon group which may have a substituent, a heterocyclic group which may have a substituent, or a group in which two or more thereof are combined, more preferably an alkyl group which may have a fluorine atom, and still more preferably a group represented by the formula (10). The alkyl group is preferably an alkyl group having 1 to 4 carbon atoms such as a methyl group, an ethyl group, a propyl group, or a butyl group, and more preferably the methyl group.

### Compound represented by formula (3) (compound 3)

The compound 3 is a compound obtained by reacting the compound 1 with the compound 2.

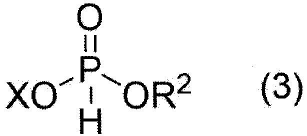

The definition of each group in the formula (3) is as described above.

### Zn catalyst

The Zn catalyst is a catalyst containing Zn (zinc).

The structure of the Zn catalyst is not particularly limited as long as it contains Zn, and the Zn catalyst may be a complex containing a ligand or a salt. In addition, the Zn catalyst may contain an organic substance. In a case where the Zn catalyst contains an organic substance, a carbon-zinc bond may be formed. Further, the Zn catalyst may be an inorganic Zn catalyst.

Above all, the Zn catalyst preferably contains a ligand from the viewpoint that the effect of the present invention is more excellent.

The ligand may be a monodentate ligand or a polydentate ligand.

The type of the ligand is not particularly limited, and examples thereof include an anionic ligand (anion) and a neutral ligand.

Specific examples of the anionic ligand include a sulfonate anion, a carboxylate anion, a phosphate anion, a monoanion having a β-diketone structure (preferably a ligand represented by the formula (B) which will be described later. Specifically, acetylacetonate and 2,2,6,6-tetramethyl-3,5-heptanedionate), an imide anion, a halide ion, and a hydroxide ion. More specifically, a trifluoromethanesulfonate anion, a salicylate anion, an acetate anion, a pivalate anion, a benzoate anion, a trifluoroacetate anion, a perchlorate anion, a bis(trifluoromethanesulfonyl)methyl anion, a bis(trifluoromethanesulfonyl)benzyl anion, a bis(trifluoromethanesulfonyl) imide anion, a chloride anion, a bromide anion, an iodide anion, a fluoride anion, and a hydroxide ion can be mentioned.

Specific examples of the neutral ligand include carbonyl, alkene, alkyne, cyclopentadienyl, benzene, cyclooctadiene, and cyclooctatetraene.

Above all, the Zn catalyst preferably contains an oxygen-containing organic ligand from the viewpoint that the effect of the present invention is more excellent. The oxygen-containing organic ligand is an organic ligand containing an oxygen atom. Examples of the oxygen-containing organic ligand include a sulfonate anion, a carboxylate anion, a phosphate anion, and a monoanion having a β-diketone structure, all of which are described above.

In addition, the Zn catalyst is preferably a catalyst represented by the formula (A), from the viewpoint that the effect of the present invention is more excellent.

Formula (A) Zn(L)₂

L represents an oxygen-containing organic ligand. The oxygen-containing organic ligand is as described above.

In addition, the Zn catalyst preferably contains a ligand represented by the formula (B) or a carboxylate anion, from the viewpoint that the effect of the present invention is more excellent. It should be noted that the ligand represented by the formula (B) corresponds to a monoanionic ligand.

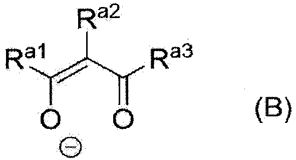

R^{a1} to R^{a3} each independently represent a hydrogen atom or an organic group.

The definition of the organic group represented by R^{a1} to R^{a3} is the same as the definition of the organic group represented by X described above.

Above all, from the viewpoint that the effect of the present invention is more excellent, R^{a1} and R^{a3} are preferably an aliphatic hydrocarbon group which may have a substituent, more preferably an alkyl group which may have a substituent, and still more preferably an alkyl group having 1 to 4 carbon atoms. R^{a2} is preferably a hydrogen atom.

Examples of the carboxylate anion include a salicylate anion, an acetate anion, a pivalate anion, a benzoate anion, and a trifluoroacetate anion.

Specific examples of the Zn catalyst include Zn(acac)₂, Zn(TMHD)₂, Zn(OAc)₂, Zn (OTf)₂, Zn(PhCO₂)₂, Zn(Salicylate)₂, Zn(Et)₂, Zn₄(TFA)₆O, and Zn(OPiv)₂.

It should be noted that acac represents acetylacetonate, TMHD represents 2,2,6,6-tetramethyl-3,5-heptanedionate, OAc represents an acetate anion, OTf represents a trifluoromethanesulfonate anion, PhCO₂ represents a benzoate anion, Salicylate represents a salicylate anion, Et represents an ethyl group, TFA represents a trifluoroacetate anion, and OPiv represents a pivalate anion.

### Other components

In the manufacturing method of the present invention, other components may be used, if necessary.

For example, the reaction between the compound 1 and the compound 2 may be carried out in the further presence of a solvent.

The type of the solvent is not particularly limited, and is preferably a solvent having a Fedors' solubility parameter (SP value) of less than 11.21 (cal/cm³)^{1/2} (hereinafter, also simply referred to as "specific solvent") from the viewpoint that the effect of the present invention is more excellent. The Fedors' solubility parameter of the specific solvent is preferably 10.00 (cal/cm³)^{1/2} or less and more preferably 9.20 (cal/cm³)^{1/2} or less. The lower limit of the Fedors' solubility parameter of the specific solvent is not particularly limited, and is preferably 7.00 (cal/cm³)^{1/2} or more and more preferably 8.00 (cal/cm³)^{1/2} or more.

Examples of the solvent having a Fedors' solubility parameter of less than 11.21 (cal/cm³)^{1/2} include a hydrocarbon, a halogenated hydrocarbon, a ketone, an ester, and an ether. More specifically, toluene (SP value: 9.14 (cal/cm³)^{1/2}), tetrahydrofuran (SP value: 8.28 (cal/cm³)^{1/2}), ethyl acetate (SP value: 8.74 (cal/cm³)^{1/2}), benzotrifluoride (SP value: 8.19 (cal/cm³)^{1/2}), and dichloromethane (SP value: 9.21 (cal/cm³)^{1/2}) can be mentioned.

It should be noted that the "Fedors' solubility parameter (SP)" in the present specification refers to a solubility parameter calculated by the so-called Fedors method described in "R.F. Fedors, Polymer Engineering Science, 14, p. 147 (1974)" or the like.

It should be noted that the solubility parameter of a mixed solvent in which a plurality of specific solvents are mixed is a value obtained by calculating a product of a solubility parameter of each specific solvent to be mixed and a mass percentage of each specific solvent with respect to the total mass of the mixed solvent, and summing the obtained values.

In addition, the reaction between the compound 1 and the compound 2 may be carried out in the further presence of an alcohol removing agent (dealcoholization agent). As will be described later, from the viewpoint that the effect of the present invention is more excellent, it is preferable to carry out the reaction while removing the alcohol compound (compound represented by the formula (4) which will be described later) produced as a by-product from the reaction system. Alcohol can be removed from the reaction system by allowing the alcohol removing agent to be present in the reaction system.

The type of alcohol removing agent is not particularly limited, and examples thereof include a molecular sieve, calcium oxide, and magnesium oxide.

The molecular sieve is a crystalline metal alumina silicate having a three-dimensional interconnected network of silica and tetrahedral alumina. The molecular sieve is capable of adsorbing various polar compounds in the cavities formed by the network. Examples of the molecular sieve include molecular sieves of different qualities such as 3A, 4A, and 5A in powder form, which can be purchased from Sigma-Aldrich Co. LLC.

### Procedure of manufacturing method

As described above, in the manufacturing method of the present invention, the compound 1 and the compound 2 are reacted in the presence of a Zn catalyst to obtain the compound 3.

The optimum reaction conditions are appropriately selected according to the type of the component used, and from an industrial point of view, it is preferable to carry out the reaction under mild conditions. More specifically, the reaction temperature is preferably 90°C or lower, more preferably 80°C or lower, still more preferably 70°C or lower, and particularly preferably 60°C or lower. The lower limit of the reaction temperature is not particularly limited, and is preferably -30°C or higher, more preferably -20°C or higher, and still more preferably 0°C or higher from the viewpoint that the effect of the present invention is more excellent.

The reaction time is preferably 0.5 to 48 hours, more preferably 2 to 30 hours, and still more preferably 2 to 24 hours from the viewpoint of product yield and economic efficiency.

The amount of each component used is not particularly limited, and an optimum amount used is selected according to the type of component.

The ratio of the molar amount of the compound 2 used to the molar amount of the compound 1 used (molar amount of compound 2 used/molar amount of compound 1 used) is not particularly limited and is often 0.1 to 10. From the viewpoint that the effect of the present invention is more excellent, the ratio is preferably 1.0 to 5.0 and more preferably 1.5 to 3.0.

In addition, the ratio of the molar amount of the Zn catalyst used to the molar amount of the compound 1 used is not particularly limited, and is often 0.01 to 1.0. From the viewpoint that the effect of the present invention is more excellent, the ratio is preferably 0.010 or more, more preferably 0.020 or more, still more preferably 0.030 or more, and particularly preferably 0.070 or more. The upper limit of the ratio is not particularly limited, and is preferably 0.2 or less and more preferably 0.15 or less.

The reaction atmosphere is not particularly limited, and may be under air or under an inert gas atmosphere, and is preferably under an inert gas atmosphere.

Specific examples of the inert gas include a nitrogen gas, an argon gas, and a mixed gas thereof.

The method of mixing each component is not particularly limited, and examples thereof include a method of mixing each component collectively and a method of mixing each component step by step.

As described above, a solvent may be used in the manufacturing method of the present invention.

Above all, from the viewpoint that the effect of the present invention is more excellent, it is preferable to carry out the reaction while removing a compound represented by the formula (4) (hereinafter, also simply referred to as "compound 4") produced as a by-product from the reaction system. The reaction between the compound 1 and the compound 2 proceeds further by carrying out the reaction while removing the compound 4 from the reaction system.

Formula (4) R¹OH

R¹ represents an alkyl group. The definition of R¹ is as described above.

It should be noted that the method of removing the compound 4 from the reaction system is not particularly limited and examples thereof include a method of carrying out the reaction in the presence of the alcohol removing agent, a method of evaporating the compound 4 using an evaporator or the like to remove it from the reaction system, and a method of trapping the compound 4 using a hydrophobic filter.

In a case where the alcohol removing agent is used, the amount of the alcohol removing agent used is not particularly limited. From the viewpoint that the effect of the present invention is more excellent, the ratio of the mass (g) of the alcohol removing agent used to the mass (g) of the compound 4 produced as a by-product is preferably 1 to 100, more preferably 3 to 50, and still more preferably 15 to 50.

By-products other than the compound 4 may be obtained in the reaction. Examples of by-products other than the compound 4 include a compound represented by the formula (5).

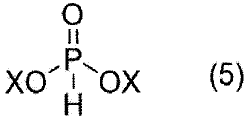

In addition, in a case where R² in the compound 3 obtained by the reaction is an alkyl group which may have a fluorine atom, the obtained compound 3 and the compound 1 may be further reacted in the presence of a Zn catalyst.

In the formula (5), X's each independently represent an organic group and have the same definition as that of X in the formula (1).

The product produced in the above steps can be separated and purified by separation means such as filtration, concentration, distillation, extraction, crystallization, recrystallization, and column chromatography, and separation means combining these.

The present invention also relates to a method for manufacturing a phosphate ester.

The method for manufacturing a phosphate ester of the present invention has a first step of reacting a compound 1 with a compound 2 in the presence of a Zn catalyst to obtain a compound 3, and a second step of reacting the compound represented by the formula (3) with a compound represented by the formula (8) (hereinafter, also simply referred to as "compound 8") in the presence of an oxidizing agent to obtain a compound represented by the formula (9) (hereinafter, also simply referred to as "compound 9").

Formula (8) X¹-OH

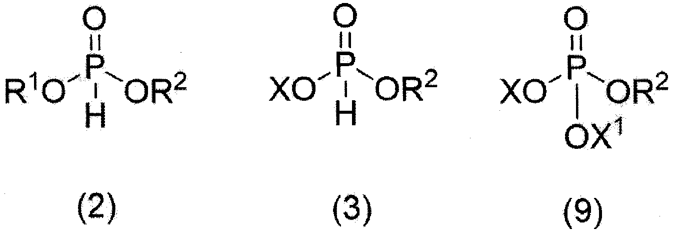

The first step corresponds to the method for manufacturing a phosphonate ester, and therefore the description thereof will be omitted.

The second step is a step of obtaining the compound 9 from the compound 3.

In the formula (8) and formula (9), X¹ represents a hydrogen atom or an organic group.

In a case where X¹ in the formula (8) is a hydrogen atom (that is, in a case where the compound 8 is water), X¹ in the formula (9) also represents a hydrogen atom. In addition, in a case where X¹ in the formula (8) is an organic group, X¹ in the formula (9) also represents the same type of organic group.

The definition of the organic group represented by X¹ is the same as the definition of the organic group represented by X described above.

Above all, from the viewpoint that the effect of the present invention is more excellent, X¹ is preferably an aliphatic hydrocarbon group which may have a substituent or a hydrogen atom, more preferably an alkyl group which may have a substituent or a hydrogen atom, and still more preferably an alkyl group having 1 to 3 carbon atoms or a hydrogen atom.

The compound 8 is water or an alcohol having an OH group. The definition of the organic group represented by X¹ in the compound 8 is as described above.

The ratio of the molar amount of the compound 3 used to the molar amount of the compound 8 used is not particularly limited and is often 0.1 to 200. From the viewpoint that the effect of the present invention is more excellent, the ratio is preferably 0.5 to 10 and more preferably 1 to 5.

The definitions of X and R² in the compound 9 are the same as the definitions of X and R² in the compound 3.

The type of oxidizing agent used in the present step is not particularly limited, and a known oxidizing agent can be used. Examples of the oxidizing agent include iodine and (10-camphorsulfonyl)oxaziridine.

The ratio of the molar amount of the oxidizing agent used to the molar amount of the compound 8 used is not particularly limited, and is often 0.1 to 10. From the viewpoint that the effect of the present invention is more excellent, the ratio is preferably 0.2 to 5 and more preferably 0.3 to 3.

In the present step, a reoxidizing agent may be used together with the oxidizing agent. Examples of the reoxidizing agent include tert-butyl hydroperoxide and hydrogen peroxide.

The ratio of the molar amount of the reoxidizing agent used to the molar amount of the compound 3 used is not particularly limited, and is often 0.1 to 100. From the viewpoint that the effect of the present invention is more excellent, the ratio is preferably 0.5 to 50 and more preferably 1 to 10.

The optimum reaction conditions for the step 2 are appropriately selected according to the type of the component used, and from an industrial point of view, the reaction temperature is preferably 0°C to 80°C and more preferably 10°C to 50°C.

The reaction time is preferably 0.5 to 48 hours, more preferably 2 to 30 hours, and still more preferably 2 to 24 hours from the viewpoint of product yield and economic efficiency.

The reaction atmosphere is not particularly limited, and may be under air or under an inert gas atmosphere, and is preferably under an inert gas atmosphere.

Specific examples of the inert gas include a nitrogen gas, an argon gas, and a mixed gas thereof.

The method of mixing each component is not particularly limited, and examples thereof include a method of mixing each component collectively and a method of mixing each component step by step.

The first step and the second step are preferably carried out in one pot. In the present invention, the one pot means that two or more reactions are carried out in the same container without undergoing an operation of distilling off the compound as an intermediate to the outside of the system.

The product produced in the above steps can be separated and purified by separation means such as filtration, concentration, distillation, extraction, crystallization, recrystallization, and column chromatography, and separation means combining these.

### Examples

Hereinafter, the present invention will be described in more detail with reference to examples, but the present invention is not limited thereto.

Identification of the compounds obtained in Examples and Comparative Examples was carried out by ¹H NMR measurement. ¹H NMR measurement was carried out using JEOL JNM-ECA 600 or ECX 500, and chloroform was used as a standard substance.

Column chromatography which will be described later was carried out using SILICA GEL 60N (available from Kanto Chemical Co., Inc., spherical, neutral).

MOLECULAR SIEVES 3A to 5A were purchased from Sigma-Aldrich Co. LLC, and dried in an environment of 200°C and lower than 1 mmHg for 72 hours or more before use.

Compounds 1a and 2a, which will be described later, were used after purchasing commercially available products and subjecting them to a distillation treatment.

### Example A1

Zn(acac)₂ (7.9 mg, 0.03 mmol) which is a Zn catalyst and MOLECULAR SIEVE 4A (100 mg) which is an alcohol removing agent were weighed in a glove box and then placed in a test tube.

Next, toluene (1 mL) was added to the test tube. Then, a compound 2a (dimethyl phosphonate) (99.0 mg, 0.90 mmol) was added to the test tube. Further, a compound 1a (cyclohexanol) (30.0 mg, 0.30 mmol) was added to the test tube, followed by reaction at 60°C for 18 hours.

The obtained product was filtered using Celite, and the obtained solution was concentrated *in vacuo* to obtain a crude product. The obtained crude product was purified by column chromatography (developing solvent: hexane/ethyl acetate = 2/1, Rf = 0.3) to obtain a compound 3a (see the scheme below). The yield of the compound 3a was 77%.

It should be noted that the ratio of the molar amount of the compound 2a used to the molar amount of the compound 1a used was 3.0, and the ratio of the molar amount of the Zn catalyst used to the molar amount of the compound 1a used was 0.1. The ratio of the mass of the alcohol removing agent used to the mass of methanol produced as a by-product was 10.

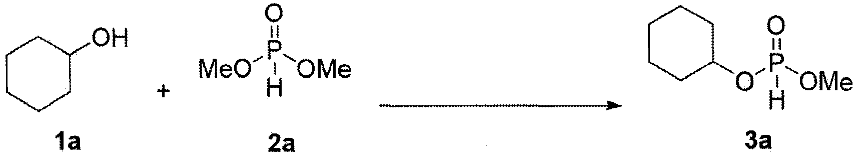

### Examples A2 to A6, and Comparative Examples 1 to 10

The compound 3a was obtained in the same manner as in Example 1, except that the type of Zn catalyst was changed as shown in Table 1 which will be described later. The results of Examples and Comparative Examples are summarized in Table 1.

In Table 1, "<5" represents less than 5.

| Table 1 | Zn catalyst | Yield (%) |
|---|---|---|
| Example A1 | Zn(acac)₂ | 77 |
| Example A2 | Zn(OAc)₂ | 46 |
| Example A3 | Zn(OTf)₂ | 56 |
| Example A4 | Zn(PhCO₂)₂ | 52 |
| Example A5 | Zn(Salicylate)₂ | 64 |
| Example A6 | Zn₄(TFA)₆O | 77 |
| Comparative Example 1 | Ti(OiPr)₄ | <5 |
| Comparative Example 2 | Mg(OAc)₂ | 8 |
| Comparative Example 3 | Sc(OTf)₃ | <5 |
| Comparative Example 4 | Yb(OTf)₃ | 10 |
| Comparative Example 5 | Hf(OTf)₄ | <5 |
| Comparative Example 6 | Fe(acac)₃ | <5 |
| Comparative Example 7 | Co(acac)₃ | <5 |
| Comparative Example 8 | Cu(TMHD)₂ | <5 |
| Comparative Example 9 | Ag(OAc) | <5 |
| Comparative Example 10 | Bi(OAc)3 | 11 |

As shown in Table 1, it was confirmed that the desired effect could be obtained in a case where a Zn catalyst was used.

### Example B1

The compound 3a was obtained in the same manner as in Example 1, except that the reaction temperature was changed from 60°C to 40°C. The yield of the compound 3a was 87%.

### Example B2

The compound 3a was obtained in the same manner as in Example B1, except that the ratio of the molar amount of the Zn catalyst used to the molar amount of the compound 1a used was changed from 0.1 to 0.05. The yield of the compound 3a was 53%.

### Example B3

The compound 3a was obtained in the same manner as in Example B1, except that the ratio of the molar amount of the Zn catalyst used to the molar amount of the compound 1a used was changed from 0.1 to 0.025. The yield of the compound 3a was 41%.

### Example B4

The compound 3a was obtained in the same manner as in Example B1, except that Zn₄(TFA)₆O was used instead of Zn(acac)₂. The yield of the compound 3a was 65%.

| Table 2 | Zn catalyst | Ratio (Zn catalyst/compound 1a) | Yield (%) |
|---|---|---|---|
| Example B1 | Zn(acac)₂ | 0.1 | 87 |
| Example B2 | Zn(acac)₂ | 0.05 | 53 |
| Example B3 | Zn(acac)₂ | 0.025 | 41 |
| Example B4 | Zn₄(TFA)₆O | 0.1 | 65 |

As shown in Table 2, it was confirmed that the effect was more excellent in a case where the ratio (Zn catalyst/compound 1a) was 0.020 or more.

In addition, from the comparison between Examples B1 and B4, it was confirmed that the effect was more excellent in a case where Zn(acac)₂ was used.

### Examples C1 to C4

The compound 3a was obtained in the same manner as in Example B3, except that the solvent shown in Table 3 was used instead of toluene. The results are summarized in Table 3.

It should be noted that, in Table 3, the yields of Examples C1 to C4 are relative values with the yield of Example B3 set to "1.00" and are shown in the column of "Yield ratio".

| Table 3 | Solvent | Fedors' solubility parameter (cal/cm³)^{1/2} | Yield ratio |
|---|---|---|---|
| Example B3 | Toluene | 9.14 | 1.00 |
| Example C1 | Tetrahydrofuran | 8.28 | 0.98 |
| Example C2 | Ethyl acetate | 8.74 | 1.20 |
| Example C3 | Acetonitrile | 11.21 | 0.44 |
| Example C4 | Benzotrifluoride | 8.19 | 1.66 |

As shown in Table 3, it was confirmed that the effect was more excellent in a case where a solvent having a Fedors' solubility parameter of less than 11.21 (cal/cm³)^{1/2} was used.

### Examples D1 to D5

The compound 3a was obtained in the same manner as in Example C4, except that the type and amount of the alcohol removing agent used were changed as shown in Table 4.

In Table 4, MS4A means MOLECULAR SIEVE 4A, MS3A means MOLECULAR SIEVE 3A, and MS5A means MOLECULAR SIEVE 5A.

| Table 4 | Alcohol removing agent | | | Yield (%) |
|---|---|---|---|---|
| | Type | Amount used (mg) | Ratio of mass of alcohol removing agent used to mass of methanol produced as by-product | |
| Example C4 | MS4A | 100 | 10 | 68 |
| Example D1 | MS3A | 100 | 10 | 76 |
| Example D2 | MS5A | 100 | 10 | 77 |
| Example D3 | MS3A | 200 | 20 | 86 |
| Example D4 | MS5A | 200 | 20 | 87 |
| Example D5 | MS5A | 400 | 40 | 88 |

As shown in Table 4, the desired effect was obtained even in a case where the type of the alcohol removing agent was changed.

### Examples E1 and E2

The compound 3a was obtained in the same manner as in Example D4, except that the ratio of the molar amount of the compound 2a used to the molar amount of the compound 1a used was changed as shown in Table 5.

| Table 5 | Ratio (compound 2a/compound 1a) | Yield (%) |
|---|---|---|
| Example D4 | 3 | 87 |
| Example E1 | 1.5 | 81 |
| Example E2 | 2 | 87 |

As shown in Table 5, the desired effect was obtained even in a case where the ratio of the molar amount of the compound 2a used to the molar amount of the compound 1a used was changed.

### Examples F1 and F2

The compound 3a was obtained in the same manner as in Example E2, except that the reaction temperature was changed as shown in Table 6.

| Table 6 | Temperature (°C) | Yield (%) |
|---|---|---|
| Example E2 | 40 | 87 |
| Example F1 | 20 | 94 |
| Example F2 | 0 | 94 |

As shown in Table 6, the desired effect was obtained even in a case where the reaction temperature was changed.

### Examples G1 to G11

A compound was obtained in the same manner as in Example F2, except that the compound 1a used was changed to the compound shown in the column of "Compound 1" in Table 7 and the reaction temperature was changed as shown in Table 7.

It should be noted that the yield shown in Table 7 represents the yield in ¹H NMR measurement in a case where a small amount of solution was sampled from the reaction solution and chloroform was used as a standard substance.

| Table 7 | Compound 1 | Compound 3 | Temperature (°C) | Yield (%) |
|---|---|---|---|---|
| Example F2 | 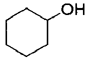 | 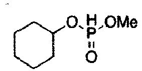 | 0 | 94 |
| Example G1 | 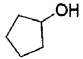 | 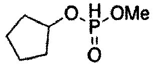 | 20 | 90 |
| Example G2 | 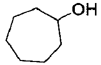 | 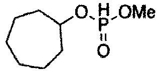 | 20 | 98 |
| Example G3 | 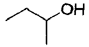 | 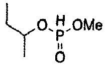 | 40 | 84 |
| Example G4 | 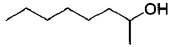 | 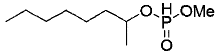 | 20 | 100 |
| Example G5 | 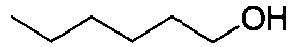 | 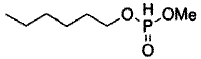 | 0 | 97 |
| Example G6 | 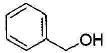 | 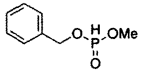 | 0 | 75 |
| Example G7 | 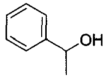 | 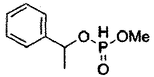 | 40 | 100 |
| Example G8 | 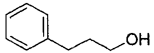 | 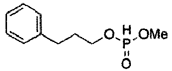 | 0 | 100 |
| Example G9 | 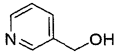 | 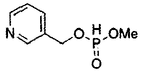 | 40 | 97 |
| Example G10 | 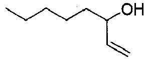 | 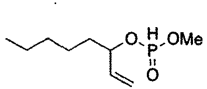 | 0 | 87 |
| Example G11 | 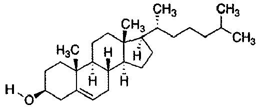 | 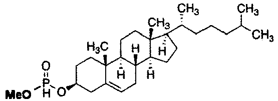 | 0 | 97 |

As shown in Table 7, the desired effect was obtained even in a case where the raw materials were changed.

### Example HI

Zn(acac)₂ (2.0 mg, 0.0075 mmol) which is a Zn catalyst and MOLECULAR SIEVE 5A (200 mg) which is an alcohol removing agent were placed in a test tube.

After purging the inside of the test tube with argon, benzotrifluoride (1 mL) was then added to the test tube. Then, a compound 2b (dimethyl phosphonate) (66.0 mg, 0.60 mmol) and a compound 1b (cyclohexanol) (30.0 mg, 0.30 mmol) were added to the test tube and reacted at room temperature (25°C) for 18 hours.

The obtained product was diluted with ethyl acetate (5 mL), the solid contents were removed by filtration from the diluted solution and washed twice with ethyl acetate (5 mL). The filtrate obtained by filtration and the washed solution of the solid contents were mixed, and the solvent was concentrated *in vacuo* to obtain a crude product. The obtained crude product was purified by column chromatography (developing solvent: hexane/ethyl acetate = 1/2) to obtain a compound 3b in the amount of 50.2 mg (see the scheme below). The yield of the compound 3b was 94%.

It should be noted that the ratio of the molar amount of the compound 2b used to the molar amount of the compound 1b used was 2.0, and the ratio of the molar amount of the Zn catalyst used to the molar amount of the compound 1b used was 0.025. The ratio of the mass of the alcohol removing agent used to the mass of methanol produced as a by-product was 20.

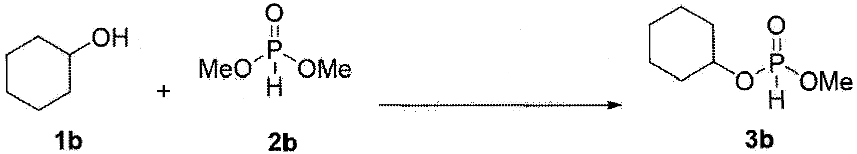

Zn(OAc)₂ (1.4 mg, 0.0075 mmol) which is a Zn catalyst and MOLECULAR SIEVE 5A (200 mg) which is an alcohol removing agent were placed in a test tube.

After purging the inside of the test tube with argon, benzotrifluoride (1 mL) was then added to the test tube. Then, a compound 3b (106.9 mg, 0.60 mmol) and a compound 4b (benzyl alcohol) (32.4 mg, 0.30 mmol) were added to the test tube and reacted at room temperature (25°C) for 18 hours.

The obtained product was diluted with ethyl acetate (5 mL), the solid contents were removed by filtration from the diluted solution and washed twice with ethyl acetate (5 mL). The filtrate obtained by filtration and the washed solution of the solid contents were mixed, and the solvent was concentrated *in vacuo* to obtain a crude product. The obtained crude product was purified by column chromatography (developing solvent: hexane/ethyl acetate = 2/1) to obtain a compound 5b (see the scheme below). The yield of the compound 5b was 88% as a result of ¹H NMR analysis.

It should be noted that the ratio of the molar amount of the compound 3b used to the molar amount of the compound 4b used was 2.0, and the ratio of the molar amount of the Zn catalyst used to the molar amount of the compound 4b used was 0.025. The ratio of the mass of the alcohol removing agent used to the mass of methanol produced as a by-product was 20.

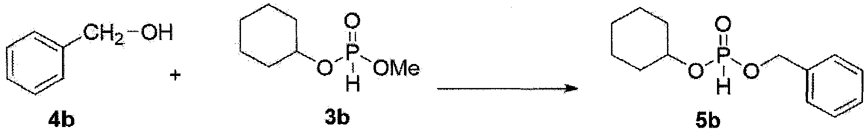

### Example I1

Zn(OPiv)₂ (2.0 mg, 0.0075 mmol) which is a Zn catalyst, a compound 1c (benzyl alcohol) (32.4 mg, 0.30 mmol), and toluene (1 mL) were placed in a flask under an argon gas atmosphere. After cooling the obtained solution to -20°C, a compound 2c (bis(2,2,2-trifluoroethyl)phosphite) (81.2 mg, 0.33 mmol) was added to the solution. The obtained solution was stirred at -20°C for 22 hours. Then, the solvent was concentrated *in vacuo* to obtain a crude product. The obtained crude product was purified by column chromatography (developing solvent: hexane/ethyl acetate = 3/1) to obtain a compound 3c (see the scheme below). The yield of the compound 3c was 93% as a result of ¹H NMR analysis, and the isolated yield was 58%.

It should be noted that the ratio of the molar amount of the compound 2c used to the molar amount of the compound 1c used was 1.1, and the ratio of the molar amount of the Zn catalyst used to the molar amount of the compound 1c used was 0.025.

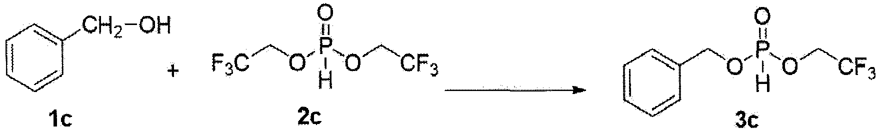

### Example I2

Zn(TMHD)₂ (3.2 mg, 0.0075 mmol) which is a Zn catalyst was placed in a test tube. After purging the inside of the test tube with argon, dichloromethane (1 mL) was then added to the test tube. Then, a compound 2d (bis(2,2,2-trifluoroethyl)phosphite) (73.8 mg, 0.30 mmol) and a compound 1d (cyclohexanol) (30.0 mg, 0.30 mmol) were added to the test tube, and the obtained solution was stirred at 0°C for 3 hours.

The solvent was concentrated *in vacuo* to obtain a crude product. The obtained crude product was purified by column chromatography (developing solvent: hexane/acetone = 5/1) to obtain a compound 3d (see the scheme below). The yield of the compound 3d was 90% as a result of ¹H NMR analysis.

It should be noted that the ratio of the molar amount of the compound 2d used to the molar amount of the compound 1d used was 1.0, and the ratio of the molar amount of the Zn catalyst used to the molar amount of the compound 1d used was 0.025.

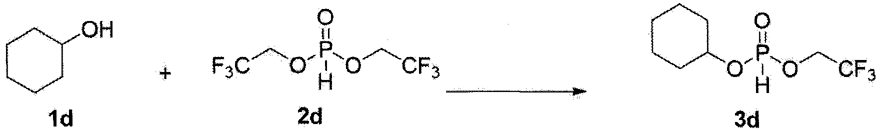

### Example J1

In a glove box, Zn(acac)₂ (2.0 mg, 0.0075 mmol) which is a Zn catalyst was placed in a test tube.

Next, toluene (1 mL) was added to the test tube. Then, a compound 2e (cyclohexyl(2,2,2-trifluoroethyl)phosphonate) (81.2 mg, 0.33 mmol) and a compound 1e (benzyl alcohol) (32.4 mg, 0.30 mmol) were added to the test tube, and the obtained solution was stirred at room temperature (25°C) for 1.5 hours.

The solvent was concentrated *in vacuo* to obtain a crude product. The obtained crude product was purified by column chromatography (developing solvent: hexane/acetone = 9/1) to obtain a compound 3e (see the scheme below). The yield of the compound 3e was 92%.

It should be noted that the ratio of the molar amount of the compound 2e used to the molar amount of the compound 1e used was 1.1, and the ratio of the molar amount of the Zn catalyst used to the molar amount of the compound 1e used was 0.025.

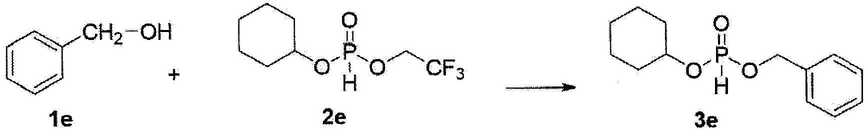

### Example J2

In a glove box, Zn(TMHD)₂ (3.2 mg, 0.0075 mmol) which is a Zn catalyst was placed in a test tube.

Next, toluene (1 mL) was added to the test tube. Then, a compound 2f (cyclohexyl(2,2,2-trifluoroethyl)phosphonate) (81.2 mg, 0.33 mmol) and a compound 1f (benzyl alcohol) (32.4 mg, 0.30 mmol) were added to the test tube, and the obtained solution was stirred at 0°C for 2 hours.

The solvent was concentrated *in vacuo,* the obtained crude product was redissolved in dichloromethane, and the obtained solution was placed in a flask. The solvent was concentrated *in vacuo* in the flask, and the inside of the flask was purged with argon. An ethanol solution (2 mL) containing iodine (40.1 mg, 0.16 mmol) and tert-butyl hydroperoxide (53.1 mg, 0.41 mmol, 70% aqueous solution) were placed in the flask, and the obtained solution was stirred at room temperature (25°C) for 12 hours. Then, a saturated aqueous sodium thiosulfate solution (1.5 mL) was placed in the flask to stop the reaction, and an extraction was carried out three times with ethyl acetate (5 mL). The obtained organic solvent was washed with brine and dried over sodium sulfate, and the solvent was concentrated *in vacuo* to obtain a crude product. The obtained crude product was purified by column chromatography (developing solvent: hexane/acetone = 5/1) to obtain a compound 3f in the amount of 64.4 mg (see the scheme below) (in the formula, Et represents an ethyl group). The yield of the compound 3f was 72%.

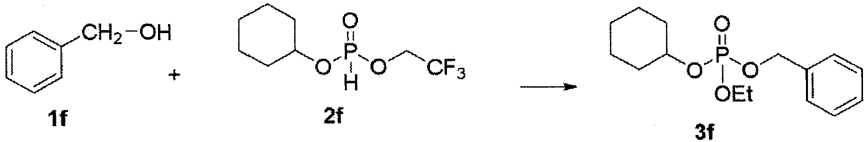

### Example K1

Zn(TMHD)₂ (4.3 mg, 0.01 mmol) which is a Zn catalyst, a compound 1 g (80.9 mg, 0.20 mmol), and MOLECULAR SIEVE 5A (200 mg) which is an alcohol removing agent were placed in a test tube.

After purging the inside of the test tube with argon, benzotrifluoride (1 mL) was then added to the test tube. Then, the test tube was cooled to 0°C, and a compound 2g (dimethyl phosphonate) (66.0 mg, 0.60 mmol) was added to the test tube, followed by reaction at 0°C for 18 hours.

The obtained product was diluted with ethyl acetate (5 mL), the solid contents were removed by filtration from the diluted solution and washed twice with ethyl acetate (5 mL). The filtrate obtained by filtration and the washed solution of the solid contents were mixed, and the solvent was concentrated *in vacuo* to obtain a compound 3g in the amount of 66.6 mg (see the scheme below). The yield of the compound 3g was 69%.

It should be noted that the ratio of the molar amount of the compound 2g used to the molar amount of the compound 1g used was 3.0, and the ratio of the molar amount of the Zn catalyst used to the molar amount of the compound 1g used was 0.05. The ratio of the mass of the alcohol removing agent used to the mass of methanol produced as a by-product was 31. In addition, in the following formula, BOM represents a benzyloxymethyl group.

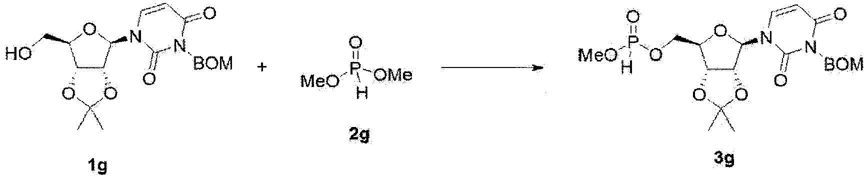

### Example K2

A compound 5g was obtained in the amount of 69.6 mg in the same manner as in Example K1, except that a compound 4g (96.7 mg, 0.20 mmol) was used instead of the compound 1g (80.9 mg, 0.20 mmol), and the amount of Zn(TMHD)₂ used was changed from 4.3 mg to 8.6 mg (see the scheme below). The yield of the compound 5g was 62%. In addition, in the following formula, Boc represents a tert-butoxycarbonyl group.

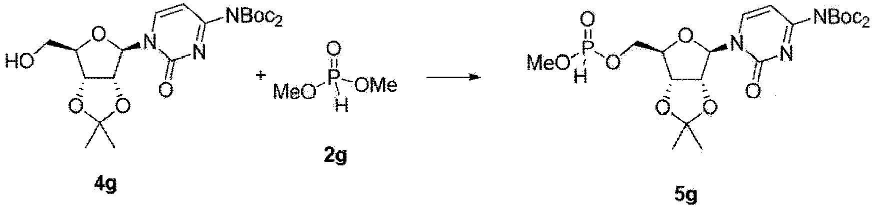

### Example K3

A compound 7g was obtained in the amount of 71.4 mg in the same manner as in Example K1, except that a compound 6g (101.5 mg, 0.20 mmol) was used instead of the compound 1g (80.9 mg, 0.20 mmol) (See scheme below). The yield of the compound 7g was 61%. In addition, in the following formula, Boc represents a tert-butoxycarbonyl group.

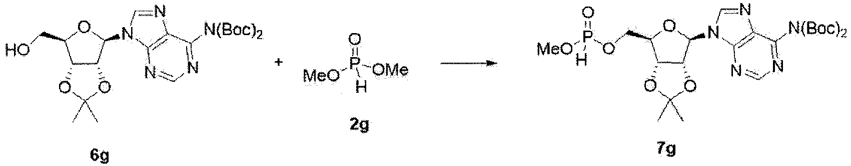

### Example K4

A compound 9g was obtained in the amount of 85.6 mg in the same manner as in Example K1, except that a compound 8g (124.7 mg, 0.20 mmol) was used instead of the compound 1g (80.9 mg, 0.20 mmol) (See scheme below). The yield of the compound 9g was 61%. In addition, in the following formula, Boc represents a tert-butoxycarbonyl group.

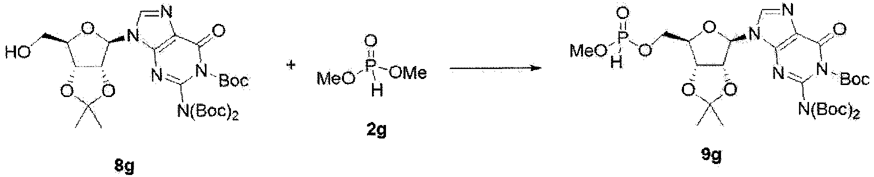

## Claims

1. A method for manufacturing a phosphonate ester, comprising:
reacting a compound represented by a formula (1) with a compound represented by a formula (2) in the presence of a Zinc catalyst to obtain a compound represented by a formula (3),
Formula (1) X-OH 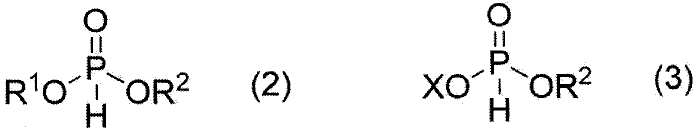
in the formulae, X represents an organic group,
R¹ represents an alkyl group that may have a fluorine atom, and
R² represents an organic group.

2. The method according to claim 1,
wherein the Zinc catalyst comprises an oxygen-containing organic ligand.

3. The method according to claim 2,
wherein the Zinc catalyst is a catalyst represented by a formula (A),
Formula (A) Zn(L)₂
in the formula, L represents the oxygen-containing organic ligand.

4. The method according to claim 1,
wherein the Zinc catalyst comprises a ligand represented by a formula (B) or a carboxylate anion, 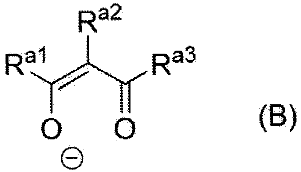
in the formula, R^{a1} to R^{a3} each independently represent a hydrogen atom or an organic group.

5. The method according to claim 1,
wherein the reaction is carried out while removing a compound represented by a formula (4), which is produced as a by-product, from a reaction system,
Formula (4) R¹OH
in the formula, R¹ represents an alkyl group.

6. The method according to claim 1,
wherein a ratio of a molar amount of the Zinc catalyst used to a molar amount of the compound represented by the formula (1) used is 0.01 or more.

7. The method according to claim 1,
wherein the reaction is carried out in the presence of a solvent having a Fedors' solubility parameter of less than 11.21 (cal/cm³)^{1/2}.

8. A method for manufacturing a phosphate ester, comprising:
a first step of reacting a compound represented by a formula (1) with a compound represented by a formula (2) in the presence of a Zinc catalyst to obtain a compound represented by a formula (3); and
a second step of reacting the compound represented by the formula (3) with a compound represented by a formula (8) in the presence of an oxidizing agent to obtain a compound represented by a formula (9),
Formula (1) X-OH
Formula (8) X¹-OH 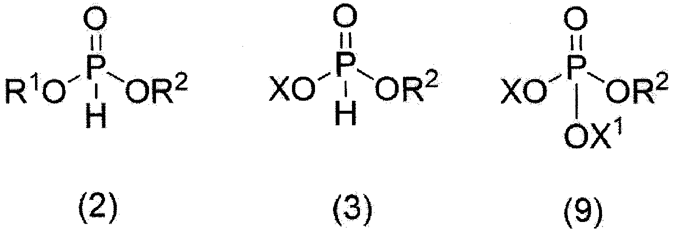
in the formulae, X represents an organic group,
X¹ represents a hydrogen atom or an organic group,
R¹ represents an alkyl group that may have a fluorine atom, and
R² represents an organic group.

9. The method for manufacturing a phosphate ester according to claim 8, wherein the first step and the second step are carried out in one pot.
